(19) Europäisches Patentamt
European Patent Office
Office européen des brevets

(11) EP 4 023 662 A1

(12) **EUROPÄISCHE PATENTANMELDUNG**

(43) Veröffentlichungstag:
**06.07.2022 Patentblatt 2022/27**

(21) Anmeldenummer: **20217861.2**

(22) Anmeldetag: **30.12.2020**

(51) Internationale Patentklassifikation (IPC):
***C07K 5/062*** *(2006.01)* ***A23K 50/80*** *(2016.01)*

(52) Gemeinsame Patentklassifikation (CPC):
**C07K 5/0606**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Evonik Operations GmbH**
**45128 Essen (DE)**

(72) Erfinder:
- **WINKLER, Tobias**
  **63457 Hanau (DE)**
- **HEIDERHOFF, Jörg**
  **50374 Erftstadt (DE)**
- **BRAUNE, Sascha**
  **2180 Antwerpen (BE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

# (54) VERFAHREN ZUR ERZEUGUNG VON PARTIKELFÖRMIGEM METHIONYL-METHIONIN

(57) Die Erfindung betrifft ein Verfahren zur Erzeugung gut filtrierbarer Partikel von DL/LD-Methionylmethionin,
dadurch gekennzeichnet, dass man einen Strom von DL/LD-Methionyl-methionin-Alkalisalz enthaltende wässrige Lösung oder Suspension so mit einer verdünnten wässrigen Mineralsäurelösung vermischt, dass innerhalb einer Mischzeit von maximal 5000 Millisekunden, vorzugsweise maximal 3000 Millisekunden eine Suspension entsteht, die einen pH-Wert von 4 bis 6 aufweist, wobei der pH-Wert gemessen wird mit Hilfe einer Glaselektrode direkt in der Suspension bei 20°C.

Dieses Verfahren ist Teil eines Gesamtverfahrens zur Herstellung von DL/LD-Methionyl-methionin, welches aus dem primär erzeugten DL/LD-Methionin-diketopiperazin durch Hydrolyse erhalten wird.

**Figur 5: Verfahrensschema zur Herstellung von DL,LD-Methionyl-methionin**

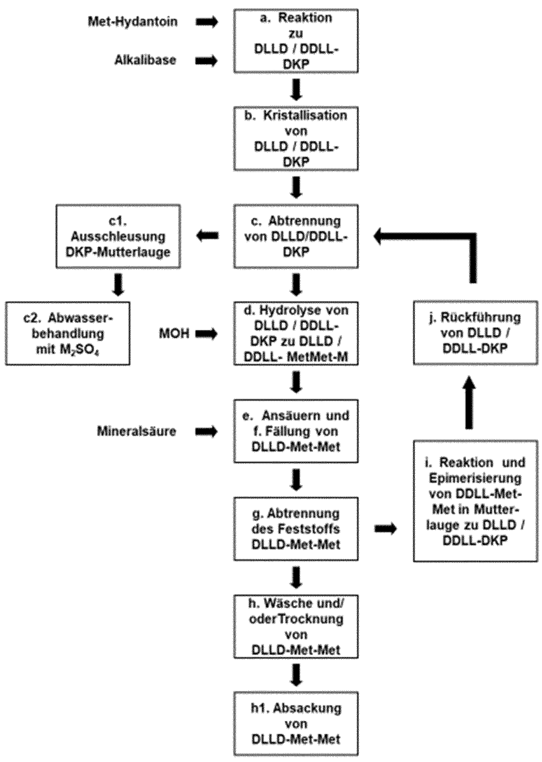


EP 4 023 662 A1

## Beschreibung

Das Dokument WO2010043558 A1 offenbart bereits ein Verfahren zur Herstellung von Methionyl-methionin (Met-Met), dem homologen Dipeptid aus DL-Methionin, welches vor allem als hochwertige Methioninquelle bei der Fütterung von in Aquakulturen gehaltenen Fischen und Krustentieren zunehmend Anwendung findet.
Die Erfindung betrifft in diesem Zusammenhang ein Verfahren zur Erzeugung gut filtrierbarer Partikel von Methionyl-methionin (Met-Met) mit der DLLD-Konfiguration.

Aufgrund seiner zwei Stereozentren (zwei asymmetrische C-Atome) liegt das Produkt in Form von zwei Diastereomeren vor, dem DL,LD-Met-Met und dem DD,LL-Met-Met, also in Form von zwei konfigurationsisomeren Enantiomerenpaaren, die zwar beide von den Tieren gut verwertet werden können, die aber deutlich unterschiedliche physikalische Eigenschaften aufweisen, was in der praktischen Handhabung zu gewissen Problemen führen kann.

Es hat sich herausgestellt, dass die beiden Diastereomeren unterschiedliche Kristallstrukturen bilden. Während DLLD-Met-Met ein kugelförmiges Agglomerat in der Endproduktfällung bildet, kristallisiert DDLL-Met-Met in Nadeln aus. Damit sind unterschiedliche Rieselfähigkeit und Verklumpungsneigung sowie unterschiedliche Löslichkeit verbunden.

Während DLLD-Met-Met bei Raumtemperatur eine Wasserlöslichkeit von nur 0,4 g/l aufweist, liegt die Wasserlöslichkeit von DDLL-Met-Met mit 21,0 g/l viel höher. Eine hohe Wasserlöslichkeit ist aber für die Anwendung in Aquakulturen eher ungünstig, da es dadurch via Leaching aus dem Futterpellet zu Wertstoffverlusten kommen kann. Das Produkt aus dem Herstellungsprozess gemäß WO2010043558 A1 weist typischerweise ein Verhältnis von DLLD-/ DDLL-Met-Met von 60 /40 auf. Ein Produkt mit deutlich höherem Anteil an DLLD-Met-Met wäre daher wünschenswert.
Im übrigen zeigt das bisher bekannte Produkt relativ niedrige Schüttgewichte von max. 500 kg/$m^3$, nur mäßige bis relative schlechte Rieselfähigkeiten von 5 (mangelhaft) bis 6 (ungenügend) und relativ niedrige Mindestzündenergie (MZE oder MIE) von < 3 mJ, ist also vergleichsweise leicht entzündlich, so dass auch diese Eigenschaften verbesserungswürdig erschienen sind.

Gemäß dem Verfahren nach WO2010043558A1 wird seit geraumer Zeit Met-Met produziert, das unter dem Produktnamen AQUAVI® Met-Met im Handel erhältlich ist.
Hierbei stellte sich die fraktionierte Kristallisation der beiden diastereomeren Enantiomerenpaare im technischen Maßstab als Schlüsselschritt für die nachfolgenden Prozessschritte dar.

Für den Anwender wichtige Produkteigenschaften sind vor allem die Fließfähigkeit und die Staubigkeit bzw. der Staubgehalt des Produkts. Eine schlechte Fließfähigkeit des Feststoffs führt beim Umfüllen oder Zudosieren ins Futtermittel zum Verblocken von Leitungen, Trichtern, Fördereinrichtungen oder anderem Equipment. Eine höherer Staubgehalt des Produkts geht aufgrund der größeren Oberfläche mit einer Absenkung der Mindestzündenergie einher. Als Staub werden unter anderem in der Sicherheitstechnik Partikel bezeichnet, die kleiner als 63 $\mu$m sind. Beides, sowohl mäßige bis schlechte Handhabungseigenschaften sowie eine Einstufung als extrem zündempfindlich aufgrund einer Mindestzündenergie unter 3 mJ, kann dazu führen, dass Hersteller von Futtermischungen ihre Anlagen anpassen bzw. aufrüsten müssen, um das Produkt überhaupt bzw. sicher fördern zu können, z. B. durch Exzonierungen. Diese zusätzlichen Investitionen können dazu führen, dass der Kunde Abstand von einem Produkt nimmt.

Die vorliegende Erfindung betrifft eine Prozessführung zur Fällung von Met-Met, die zu einem kristallinen Feststoff führt, der sehr gute Handhabungseigenschaften mit einem stark reduzierten Staubanteil aufweist.

Bei der "Kristallisation" des weniger löslichen Enantiomerenpaars, dem DLLD-Met-Met handelt es sich nicht um eine Kristallisation im klassischen Sinne, sondern um eine Fällung. Fällungen lassen sich nicht mit den Regeln einer Kristallisation beschreiben.
Die Fällung des DLLD-Met-Mets wird durch eine pH-Justierung auf einen pH von ca. 5 bei einer Temperatur oberhalb von 50 °C induziert, was dem isoelektrischen Punkt des Dipeptids entspricht. Dies kann, wie in o.g. WO-Anmeldung beschrieben, durch Erhöhen des pH-Werts einer Ammoniumsalzlösung von Met-Met mit Base oder durch Absenken des pH-Werts durch Zugabe von Säure erreicht werden. Allerdings schwankt die Qualität der Partikel stark in Abhängigkeit von der Prozessführung. So führt eine direkte Zugabe von Schwefelsäure in eine konzentrierten Lösung des Natriumsalzes des Dipeptids (NaMetMet) zu einem sehr feinen Niederschlag, der sich nur schwer abtrennen und fördern lässt.

**Aufgabe** der vorliegenden Erfindung war es, insbesondere einen robusten, kontinuierlich durchführbaren Fällungsprozess für Met-Met innerhalb eines Herstellverfahrens für Met-Met bereitzustellen, der zu einem Produkt führt, welches die Nachteile des bisherigen Produkts wie Staubigkeit und niedrige Mindestzündenergie nicht mehr zeigt und darüber hinaus gute Handhabungseigenschaften wie eine gute Rieselfähigkeit aufweist.

Überraschenderweise wurde nun gefunden, dass sich sehr schöne Agglomerate des DLLD- Met-Mets erzeugen lassen, wenn die Alkali-Met-Met-Lösung in einer auf pH 2 bis 3 eingestellten, verdünnten Säure, wie Schwefelsäure (oder rezyklierter Mutterlauge) drastisch verdünnt wird und der pH dabei unter weiterer Addition der Alkali-Met-Met-Lösung auf ca. pH 5 angehoben wird. Ist pH 5 erreicht, muss ein weiterer pH-Wertanstieg durch gleichzeitige Zugabe von Säure, in diesem Fall Schwefelsäure, ausgeglichen werden. Ein Überschießen des pH-Werts über pH 6 oder eine umgekehrte Reaktionsführung führt unweigerlich zu einem sehr feinen und schwer filtrierbaren Feststoff und muss vermieden werden. So lange sich der pH-Wert in einem Bereich zwischen 4 und 6 bewegt, sind keine bzw. geringere

Auswirkungen auf die Produktqualität zu beobachten. Dies hat zur Folge, dass an das Einmischen der Säure besondere Anforderungen gestellt werden.

Dagegen hat die Art und Weise des Einmischens der Alkali-Met-Met-Lösung, die selbst einen pH-Wert von 11 bis 14, in der Regel von ca.12 aufweist, einen entscheidenden Einfluss auf den Erfolg der Fällung. Wenn z.B. die Vermischung der Alkali-Met-Met-Lösung nicht optimal erfolgt, beispielsweise dann wenn das Alkali-Met-Met-Salz zu langsam den pH-Bereich von ca.pH 12 nach ca. pH 5 durchläuft, dann werden sehr feine bis hin zu amorphen Partikeln generiert. Dies lässt sich im Nachhinein nicht mehr korrigieren. Etwa würde ein Auflösen durch Zugabe von Natronlauge über eine anteilig stattfindende Peptidspaltung auch zu einer unerwünschten Erhöhung des Methioningehalts führen, welcher nach einer zweiten Kristallisation nicht mehr ausreichend abzusenken wäre.

Gegenstand der vorliegenden Erfindung ist somit ein Verfahren zur Erzeugung gut filtrierbarer Partikel von DL/LD-Methioninylmethionin (Ia + Ib),

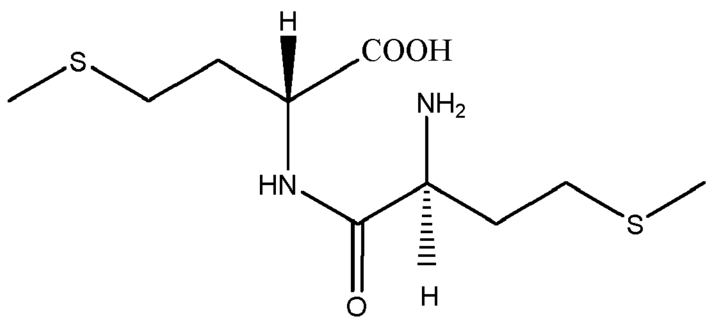

(I a) + Enantiomer (I b)

dadurch gekennzeichnet, dass man einen Strom von DL/LD-Methionyl-methionin-Alkalisalz enthaltende wässrige Lösung oder Suspension, die typischerweise einen pH-Wert von 11 bis 14 aufweist, so mit einer verdünnten wässrigen Mineralsäurelösung, die typischerweise einen pH-Wert von 1 bis 4 aufweist, vermischt, dass innerhalb einer sehr kurzen Mischzeit von maximal 5000 Millisekunden, vorzugsweise maximal 3000 Millisekunden eine Suspension entsteht, die einen pH-Wert von 4 bis 6 aufweist, wobei der pH-Wert jeweils gemessen wird mit Hilfe einer Glaselektrode direkt in der jeweiligen Lösung oder Suspension bei 20°C. Die Mischzeit ergibt sich dabei aus der Länge der Mischstrecke, also z.B. der Rohrlänge in m zwischen Mischpunkt und Reaktor und der Durchflussgeschwindigkeit des gemischten Mediums in $m^3/h$ bei gegebenem Querschnitt der Mischstrecke (z.B. Rohrquerschnitt) in $m^2$.

Besonders geeignet ist dabei ein Verfahren, dadurch gekennzeichnet, dass die homogene Lösung oder Suspension mit Hilfe eines Intensivmischers erzeugt wird.

Als Intensivmischer verwendet werden können beispielsweise T-Mischer (vgl. Figur 1), Y-Mischer, Statikmischer, Jetmischer oder Umwälzpumpen und zwar insbesondere solche, welche ungelöste Feststoffe tolerieren.

Dabei wird bevorzugt so verfahren, dass die entstandene homogene Lösung oder Suspension einen pH-Wert von 4,5bis 5,5, vorzugsweise von 4,8 bis 5,2 aufweist.

Des Weiteren bevorzugt ist hier ein Verfahren, bei dem das auf die Konzentration normierte Verhältnis $SR_m$ von Umlaufgeschwindigkeit der Suspension (bulk) zu Zuflussgeschwindigkeit (Alkali-Met-Met) von 5 bis 17, vorzugsweise von 7 bis 15 reicht. Auf diese Weise gelingt es, ein vergleichsweise grobes und somit besser handhabbares Produkt mit einem Median der Korngrößenverteilung $X_{50}$ von etwa 100 bis 350 $\mu$m zu erhalten (vgl. Figur 3).

Besonders günstig erwiesen haben sich sehr kurze Mischzeiten im Bereich von 500 bis 2500 Millisekunden.

Um diese Einmischung optimal gestalten zu können, konnte mit der vorliegenden Erfindung nachgewiesen werden, dass die Impulse der zu mischenden Flüssigkeiten, also Alkali-Met-Met-Lösung und auf pH-justierte Met-Met-Suspension in dem richtigen Verhältnis stehen müssen. Dabei ist es unerheblich, ob die Einmischung direkt im Reaktor oder über einen T- Mischer in der Kreislaufleitung erfolgt. Letztere Variante hat den Vorteil, dass sich die Impulsverhältnisse leichter anpassen bzw. bei unterschiedlichen Kapazitäten variieren lassen.

Im Vergleich zur Veröffentlichung von H. Rehage et al. (Chemical Engineering Science 207 (2019), 258 ff.), der die Fällungsreaktion zwischen Bariumchloridlösung und Natriumsulfatlösung untersucht hat, konnte darüber hinaus gezeigt werden, dass im vorliegenden Fall die Mischgüte weiter gesteigert werden kann, wenn die zu mischenden Ströme im T-Mischer aus entgegengesetzter Richtung aufeinander zugeführt werden (vgl. Figur 1), und nicht, wie in der Veröffentlichung aufgeführt, in einem rechten Winkel dosiert werden. Im Falle von Met-Met führte die Dosierung in einem T-Mischer mit dieser rechtwinkligen Ausrichtung analog zu Rehage nicht zu einer ausreichenden Kristallqualität.

Bevorzugt wird daher ein Verfahren, das dadurch gekennzeichnet ist, dass im Falle der Verwendung eines T-Mischers oder Y-Mischers der Anströmwinkel, in dem die beiden Ströme aufeinandertreffen 120 bis 180°, vorzugsweise 150 bis 180°, insbesondere etwa 180° beträgt. Des Weiteren bevorzugt wird im erfindungsgemäßen als verdünnte Mineralsäurelösung wässrige Schwefelsäure-, Salzsäure- oder Phosphorsäurelösung verwendet, vorzugsweise wässrige Schwefelsäurelösung, z.B. 2N Schwefelsäure.

Außerdem wird als DL/LD-Methionyl-methionin-Alkalisalz vorzugsweise das Natriumsalz oder Kaliumsalz, insbesondere das Natriumsalz des DL/LD-Methionyl-methionins eingesetzt.

Das erfindungsgemäße Verfahren kann in vorteilhafter Weise sowohl im batch als auch kontinuierlich durchgeführt werden.

Nur unter Anwendung dieser neu gewonnenen Erkenntnisse (pH- Führung, Qualität der Einmischung) ist es möglich, ein gut filtrierbares Produkt DLLD-Met-Met (AQUAVI®Met-Met) zu fällen, das sich im weiteren Verlauf leicht waschen und trocknen lässt. Die Neigung zum Verklumpen ist durch die Kristallgröße und -form und die erzielbaren Restfeuchten nach der fest/flüssig- Trennung signifikant reduziert. Das verkaufsfertige Produkt zeigt darüber hinaus noch eine höhere Schüttdichte und einen deutlich reduzierten Staubanteil, was die Handhabungseigenschaften wie etwa die Rieselfähigkeit, die Staubigkeit signifikant verbessert. Außerdem zeigt es hohe mechanische Festigkeit (vgl. Langzeitrührversuch in Beispiel 4, Figur 4).

**Gesamtverfahren zur Herstellung gut filtrierbarer DLLD-Met-Met- Partikel**

Ein weiterer Gegenstand der vorliegenden Erfindung ist das folgende Gesamtverfahren (vgl. Figur 5) zur Gewinnung von diastereomerenreinem DL/LD-Methionylmethionin, dadurch gekennzeichnet, dass man

a. Methionin-Hydantoin zusammen mit Alkalibase umsetzt zu einem Diastereomerengemisch aus DL/LD-Methionindiketopiperazin und DD/LL-Methionindiketopiperazin (DLLD-DKP und DDLL-DKP)

b. das DLLD-DKP und DDLL-DKP aus a. durch Einengen oder Abkühlen der Reaktionslösung aus a. zur Kristallisation bringt

c. das auskristallisierte DLLD-DKP und DDLL-DKP abtrennt von der DKP-Mutterlauge

d. das abgetrennte DLLD-DKP und DDLL-DKP in Wasser einmischt und das Gemisch durch Zugabe entsprechender Mengen MOH (M=Alkali) auf einen pH-Wert von 10 bis 14, vorzugsweise 13, gemessen mit einer pH-Elektrode bei 20 °C, bringt und bei einer Temperatur von 90 bis 150 °C und einer Verweilzeit von 30 bis 180 min zu einer entsprechenden wässrigen Lösung oder Suspension eines Diastereomerengemisches aus DL/LD-Methionylmethionin- und DD/LL-Methionylmethionin-Alkalisalz hydrolysiert,

e. den pH-Wert der Lösung oder Suspension aus d., gemessen bei 20°C, durch erfindungsgemäßes Vermischen mit entsprechender Mengen Mineralsäure gemäß dem oben beschriebenen erfindungsgemäßen Verfahrens auf 4 bis 6, vorzugsweise auf 4,5 bis 5,5, besonders bevorzugt auf 4,8 bis 5,2 absenkt (Ansäuern),

f. durch Verdampfen von Wasser und/ oder Abkühlen eine Suspension erzeugt (Fällung), die aus einem Feststoffanteil, der vorwiegend DL/LD-Methionylmethionin enthält, und Mutterlauge, die vorwiegend DD/LL-Methionylmethionin enthält, besteht, man

g. den Feststoffanteil aus f. von der Mutterlauge abtrennt, und man

h. den abgetrennten Feststoffanteil aus g. wahlweise wäscht und/oder trocknet, und dabei diastereomerenreines DL/LD-Methionylmethionin erhält und man

i. die verbleibende DD/LL-Methionylmethionin enthaltende Mutterlauge aus g. mittels Cyclisierung und Epimerisierung gemäß der in der parallelen Anmeldung EP20217816.6 (welche inhaltlich per Referenz einbezogen wird) beschriebenen Verfahrensweise (vgl. auch dortigen Anspruch 1) umsetzt, so dass eine wässrige Lösung oder Suspension enthaltend ein Diastereomerengemisch aus DL/LD-Methionindiketopiperazin und DD/LL-Methionindiketopiperazin entsteht mit einem Anteil von bis zu 60 Mol% DL/LD-Methionindiketopiperazin am Gesamtgehalt der DL/LD- und DD/LL-Methionyl-methioninäquivalente im Reaktionsgemisch und

j. diese dann in Schritt b. zurückführt.

Unter "diastereomerenrein" oder auch "im wesentlichen diastereomerenrein" wird in diesem Zusammenhang ein Produkt verstanden, das zumindest 90 Mol% des gewünschten DL/LD-Diastereomeren enthält, vorzugsweise mindestens 95 bis 97 Mol% am Gesamtgehalt an enthaltenem Methionyl-methionin (Met-Met).

**Figur 5 zeigt das Schema zur Herstellung DL/LD-Methionyl-methionins**

Das Schema umfasst die folgenden Schritte:

a. Reaktion von Methioninhydantoin mit Alkalibase zu DLLD/DDLL-Met-DKP
b. Kristallisation von DLLD/DDLL-Met-DKP
c. Abtrennung von DLLD/DDLL-Met-DKP

c1. Ausschleusung von DKP-Mutterlauge
c2. Abwasserbehandlung

d. alkalische Hydrolyse von DLLD/DDLL-Met-DKP zu DLLD/DDLL-Met-Met
e. Ansäuern und
f. Fällung von DLLD-Met-Met
g. Abtrennung des Feststoffs DLLD -Met-Met
h. Wäsche und/oder Trocknung von DLLD-Met-Met
h1. Absackung von DLLD-Met-Met
i. Reaktion und Epimerisierung von DDLL -Met in DDLL -Met-haltiger Mutterlauge zu DLLD/DDLL-Met-DKP
j. Rückführung von DLLD/DDLL-Met-DKP in Schritt c.

**Beispiele**

Die angeführten Beispiele wurden in einer technischen Versuchsanlage durchgeführt.

**Definitionen und Methoden**

SR (Speed Ratio, Geschwindigkeitsverhältnis) [-] =
Umlaufgeschwindigkeit der Suspension im Reaktor [cbm/h]/ Zuflussgeschwindigkeit [cbm/h]

$SR_m$ (Speed Ratio modified, Geschwindigkeitsverhältnis modifiziert) [1/Gew.%] =
Umlaufgeschwindigkeit der Suspension im Reaktor [cbm/h]/ Zuflussgeschwindigkeit
[cbm/h*]*NaMetMet-Konzentration [Gew.%].

Die **Korngrößenverteilung** der Partikel in der Suspension wurde mittels eines Horiba LA 350 Laserstreulichtspektrometers im Messbereich von 0,1 bis 1000 μm bestimmt.

**Bestimmung der Schüttdichte**

Die Schüttdichte in kg/L wurde bestimmt mit Hilfe eines 1 L Messzylinders, der mit dem Schüttgut genau bis zum Marke 1000 ml aufgefüllt wurde und per Wägung das Gewicht des Inhalts gemessen wurde, so dass sich damit direkt die Schüttdichte ergab.

**Bestimmung des Fließverhaltens**

Das Fließverhalten (Rieselfähigkeit) wurde durch Vergabe einer Rieselnote von 1 (sehr gut) bis 6 (ungenügend) bestimmt aufgrund vorheriger Messung der Rieselfähigkeit mit Hilfe von Standardauslaufgefäßen aus Glas. Diese hatten eine zylindrische Form mit konischem unteren Ende, in dessen Mitte eine Auslauföffnung positioniert war (analog wie bei einem Silo) mit unterschiedlicher Weite von eng für Note 1 bis relativ weit für Note 6. Die Messung wurde mit dem engsten Auslaufgefäß begonnen, die Auslauföffnung mit einem Glasplatte zugehalten, das Auslaufgefäß mit dem Schüttgut befüllt, die Glasplatte weggenommen und das Auslaufen beurteilt. Nur wenn ein praktisch ungehemmtes Auslaufen das Schüttgutes beobachtet werden konnte, dann wurde die Note 1 gegeben. Wenn dies nicht der Fall war wurde das

nächstweitere Auslaufgefäß verwendet und bei einwandfreiem Auslaufen die Note 2 vergeben, wenn dies wiederum nicht der Fall war wurde das nächst weitere Auslaufgefäß verwendet usw. bis zum weitesten Auslaufgefäß (Gefäß 5). Nur Material, das auch aus diesem Auslaufgefäß nicht ungehemmt ausfließen konnte, wurde mit Note 6 bewertet. Die Standardauslaufgefäße hatten alle eine Höhe des zylindrischen Teils von 70 mm und einen innere Weite von 36 mm. Die Auslaufsöffnungen hatten die folgenden Weiten für Gefäß1: 2,5 mm, Gefäß 2: 5 mm, Gefäß 3: 8 mm, Gefäß 4: 12mm, Gefäß 5: 18 mm.

**Bestimmung des pH-Wertes**

Der pH-Wert wurde mit Hilfe einer Glaselektrode in wässriger Lösung und bei einer Temperatur von 20°C gemessen sofern nicht anders angegeben.

Die **Quantitative Bestimmung der Konzentrationen** von Met, Met-Met, Methionyl-DKP usw. erfolgte mit Standard-HPLC Methoden gegenüber einem externen Standard.

**Beispiel 1** (Konventionelles Batchverfahren zur Fällung der Met-Met-Partikel)

Eine im Verfahren zur Herstellung von Met-Met gemäß WO2010043558A1 primär erzeugte NaMet-Met-Suspension (mit 30 Gew.% Met-Met) wurde mit einer Umlaufgeschwindigkeit zwischen 35 und 50 cbm/h in einem Rührreaktor mit Schlaufe (wie in Figur 2 abgebildet) im Kreis gefahren. Dabei erfolgte die Mischung der NaMet-Met-Lösung und der mit Schwefelsäure versetzten Kreislauflösung (Zugabe von verdünnter Schwefelsäure mit pH 2 - 3 in die Zirkulationsleitung) direkt im Rührreaktor, der als Misch- und Verweilzeitbehälter Teil des gesamten Schlaufenreaktors war. Dabei erfolgte kein Austrag der entstandenen Produktmischung während der Reaktion, sondern der Reaktor wurde bis zum maximalen Füllstand gefahren und danach eine Probe gezogen zur Analyse der Korngrößenverteilung. Die Ergebnisse in Form der Median-Werte X50 der Korngrößenverteilung sind in Figur 3 aufgetragen.

**Beispiel 2** (T-Mischer im Batchverfahren zur Fällung der Met-Met-Partikel)

Eine im Verfahren zur Herstellung von Met-Met gemäß WO2010043558A1 primär erzeugte NaMet-Met-Suspension (mit 30 Gew.% Met-Met) wurde mit einer Umlaufgeschwindigkeit zwischen 35 und 50 cbm/h in einem Rührreaktor mit Schlaufe (wie in Figur 2 abgebildet) im Kreis gefahren. Dabei erfolgte die Mischung der NaMet-Met-Lösung und der mit Schwefelsäure versetzten Kreislauflösung (Zugabe von verdünnter Schwefelsäure mit pH 2 - 3 in die Zirkulationsleitung) über eine T-Mischer (analog Figur 1) oberhalb des Rühr-Reaktors, von wo aus das Gemisch direkt in den Rührreaktor eingetragen wurde. Dabei erfolgte kein Austrag der entstandenen Produktmischung während der Reaktion, sondern der Reaktor wurde bis zum maximalen Füllstand gefahren und danach eine Probe gezogen zur Analyse der Korngrößenverteilung. Die Ergebnisse in Form der Median-Werte X50 der Korngrößenverteilung sind in Figur 3 aufgetragen.

**Beispiel 3** (T-Mischer im kontinuierlichen Verfahren zur Fällung der Met-Met-Partikel)

In Beispiel 3 wurde verfahren wie in Beispiel 2, allerdings erfolgte ein fortlaufender Austrag der entstandenen Produktmischung während der Reaktion, so dass der Füllstand im Reaktor während der Dauer der Reaktion in etwa gleich gehalten wurde und aus dem kontinuierlichen Austragsstrom wurde nach ca. einem Tag Laufzeit entsprechend eine Probe gezogen zur Analyse der Korngrößenverteilung. Die Ergebnisse in Form der Median-Werte $X_{50}$ der Korngrößenverteilung sind in Figur 3 aufgetragen.

**Beispiel 4** (Mechanische Stabilität der Met-Met-Partikel)

Eine wässrige Suspension von ca. 15 Gew.% DLLD-Met-Met wurde 34 h lang in einem technischen Rührreaktor (Fig.2) kontinuierlich mit einer Rührgeschwindigkeit von 60 U/min gerührt ohne Zirkulation über den Wärmeaustauscher. Zu Beginn und am Ende des Rührversuches wurde je eine Probe genommen und die Korngrößenverteilung der Partikel in der Suspension mittels eines Horiba LA 350 Laserstreulichtspektrometers im Messbereich von 0,1 bis 1000 µm bestimmt. Die beiden ermittelten Korngrößenverteilungen weisen jeweils ein Maximum bei über 100 µm auf und zeigen keine signifikanten Unterschiede, was die hohe mechanische Stabilität der erfindungsgemäßen Partikel verdeutlicht (Fig.4).

## Patentansprüche

1. Verfahren zur Erzeugung gut filtrierbarer Partikel von DL/LD-Methionyl-methionin, **dadurch gekennzeichnet, dass** man einen Strom von DL/LD-Methionyl-methionin-Alkalisalz enthaltende wässrige Lösung oder Suspension so mit einer verdünnten wässrigen Mineralsäurelösung vermischt, dass innerhalb einer Mischzeit von maximal 5000 Millisekunden, vorzugsweise maximal 3000 Millisekunden eine Suspension entsteht, die einen pH-Wert von 4 bis 6 aufweist, wobei der pH-Wert gemessen wird mit Hilfe einer Glaselektrode direkt in der Suspension bei 20°C.

2. Verfahren gemäß Anspruch 1, **dadurch gekennzeichnet, dass** die Suspension mit Hilfe eines Intensivmischers erzeugt wird.

3. Verfahren gemäß Anspruch 2, **dadurch gekennzeichnet, dass** als Intensivmischer ein T-Mischer, ein Y-Mischer, ein Statikmischer, ein Jetmischer oder eine Umwälzpumpe verwendet wird.

4. Verfahren gemäß einem der Ansprüche 1 bis 3, **dadurch gekennzeichnet, dass** die entstandene Suspension einen pH-Wert von 4,5 bis 5,5, vorzugsweise von 4,8 bis 5,2 aufweist.

5. Verfahren gemäß einem der Ansprüche 1 bis 4, **dadurch gekennzeichnet, dass** das Verhältnis $SR_m$ von Umlaufgeschwindigkeit (bulk) zu Feedgeschwindigkeit (Alkali-Met-Met) bezogen auf die Alkali-Met-Met-Konzentration einen Wert von 5 bis 17 erreicht, vorzugsweise von 7 bis 15.

6. Verfahren gemäß einem der Ansprüche 1 bis 5, **dadurch gekennzeichnet, dass** die Mischzeit im Bereich von 500 bis 2500 Millisekunden liegt.

7. Verfahren gemäß einem der Ansprüche 3 bis 6, **dadurch gekennzeichnet, dass** im Falle der Verwendung eines T-Mischers oder Y-Mischers der Anströmwinkel, in dem die beiden Ströme aufeinandertreffen 120 bis 180°, vorzugsweise 150 bis 180°, inbesondere etwa 180° beträgt.

8. Verfahren gemäß einem der Ansprüche 1 bis 7 **dadurch gekennzeichnet, dass** man als verdünnte Mineralsäurelösung wässrige Schwefelsäure-, Salzsäure- oder Phosphorsäurelösung verwendet, vorzugsweise wässrige Schwefelsäurelösung.

9. Verfahren gemäß einem der Ansprüche 1 bis 8 **dadurch gekennzeichnet, dass** man als DL/LD-Methionyl-methionin-Alkalisalz das Natriumsalz oder Kaliumsalz, vorzugsweise das Natriumsalz des DL/LD-Methionyl-methionins einsetzt.

10. Verfahren gemäß einem der Ansprüche 1 bis 9, **dadurch gekennzeichnet, dass** das Verfahren im batch oder kontinuierlich durchgeführt wird.

11. Verfahren zur Gewinnung von diastereomerenreinem DL/LD-Methionylmethionin, **dadurch gekennzeichnet, dass** man

    a. Methionin-Hydantoin zusammen mit Alkalibase umsetzt zu einem Diastereomerengemisch aus DL/LD-Methionindiketopiperazin und DD/LL-Methionindiketopiperazin (DLLD-DKP und DDLL-DKP)
    b. das DLLD-DKP und DDLL-DKP aus a. durch Einengen oder Abkühlen der Reaktionslösung aus a. zur Kristallisation bringt
    c. das auskristallisierte DLLD-DKP und DDLL-DKP abtrennt von der DKP-Mutterlauge
    d. das abgetrennte DLLD-DKP und DDLL-DKP in Wasser einmischt und das Gemisch durch Zugabe entsprechender Mengen Alkali auf einen pH-Wert von 10 bis 14, vorzugsweise 13, gemessen mit einer pH-Elektrode bei 20 °C, bringt und bei einer Temperatur von 90 bis 150 °C und einer Verweilzeit von 30 bis 180 min zu einer entsprechenden wässrigen Lösung oder Suspension eines Diastereomerengemisches aus DL/LD-Methionylmethionin- und DD/LL-Methionylmethionin-Alkalisalz hydrolysiert,
    e. den pH-Wert der Lösung oder Suspension aus d., gemessen bei 20°C, durch Vermischen mit entsprechender Mengen Mineralsäurelösung gemäß mindestens einem der Ansprüche 1 bis 10 auf pH 4 bis 6, vorzugsweise auf 4,5 bis 5,5, besonders bevorzugt auf 4,8 bis 5,2 absenkt,
    f. durch Verdampfen von Wasser und/ oder Abkühlen eine Suspension erzeugt, die aus einem Feststoffanteil, der vorwiegend DL/LD-Methionylmethionin enthält, und Mutterlauge, die vorwiegend DD/LL-Methionylmethionin enthält, besteht, man

g. den Feststoffanteil aus f. von der Mutterlauge abtrennt, und man

h. ihn wahlweise wäscht und/oder trocknet, und dabei diastereomerenreines DLLD-Methionylmethionin erhält und man

i. die verbleibende DD/LL-Methionylmethionin enthaltende Mutterlauge aus g. mittels Cyclisierung und Epimerisierung umsetzt, so dass eine wässrige Lösung oder Suspension enthaltend ein Diastereomerengemisch aus DL/LD-Methionindiketopiperazin und DD/LL-Methionindiketopiperazin entsteht mit einem Anteil von bis zu 60 Mol% DL/LD-Methionindiketopiperazin am Gesamtgehalt der DL/LD- und DD/LL-Methionyl-methioninäquivalente im Reaktionsgemisch und

j. diese dann in Schritt b. zurückführt

**Figur 1: Zeichnung eines typischen T-Mischers (a) und Y-Mischers (b) *)**

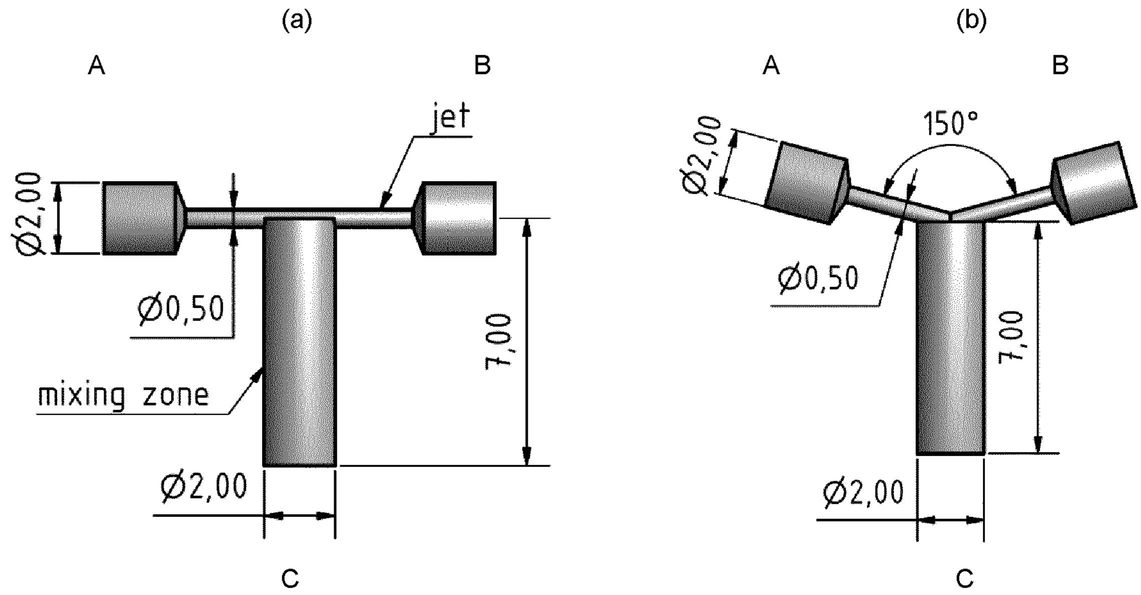


A: Einlass für Komponente A (Säure zur Acidifizierung)

B: Einlass für Komponente B (Basische Alkali-Met-Met-Lösung)

C: Auslass für Komponente C (Neutralisierte Met-Met-Lösung oder Suspension)

Mixing zone: Mischstrecke

***)** Figur 1 wurde entnommen aus Internet-Publikation „Fällungskristallisation von Katalysatorvorstufen“ des Instituts für Thermische Verfahrenstechnik (KIT), https://www.tvt.kit.edu//21_3139.php

**Figur 2: Schlaufenreaktor für batch- und kontinuierliche Fahrweisen**

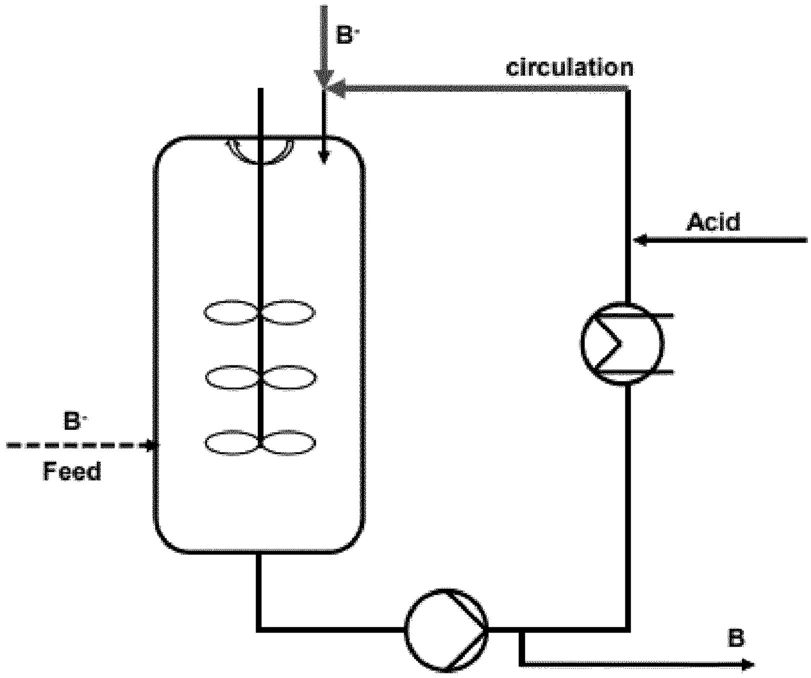


Verschiedene Reaktorfahrweisen:

Konventioneller Batch:
Zufluss der basischen Alkali-Met-Met-Lösung B- über Seitenstrom in Reaktor (unterbrochene Linie)

T-Mixer Batch:
Zufluss der basischen Alkali-Met-Met-Lösung B- im Batch-Modus über T-Mixer in Zirkulationsleitung

T-Mixer kontinuierlich:
Zufluss der basischen Alkali-Met-Met-Lösung B- kontinuierlich über T-Mixer in Zirkulationsleitung sowie kontinuierlicher Abfluss der neutralisierten Produkt-Lösung B

Figur 3: Median $X_{50}$ der erhaltenen Korngrößenverteilung bei kontinuierlichen und batch-Fahrweisen (Beispiel 1, 2 und 3) in Abhängigkeit vom $SR_m$-Wert

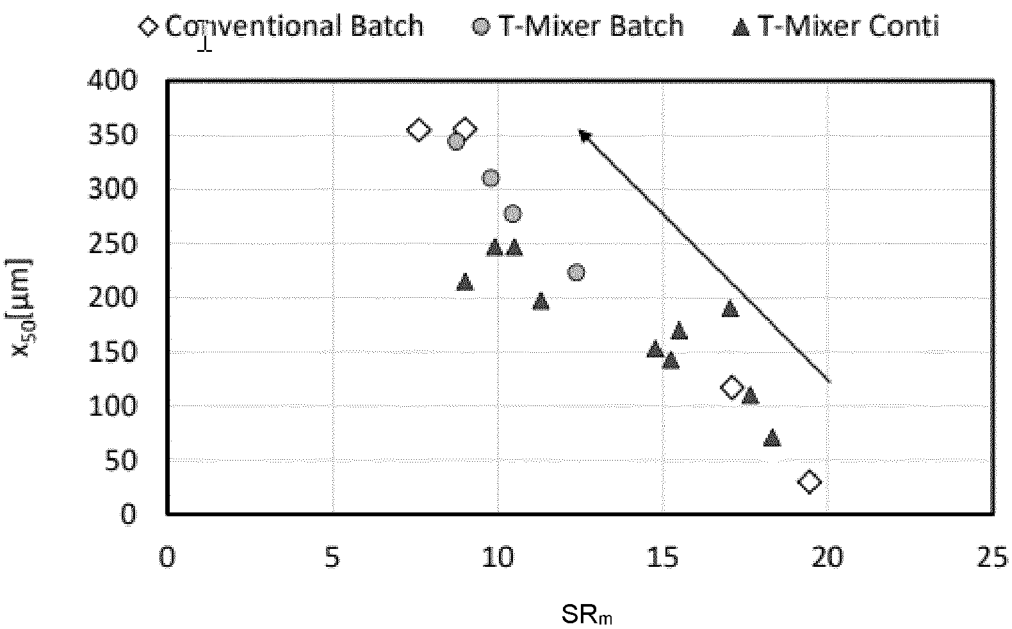

Figur 4: Korngrößenverteilungen vor und nach Rühren für 34 h (Beispiel 4)

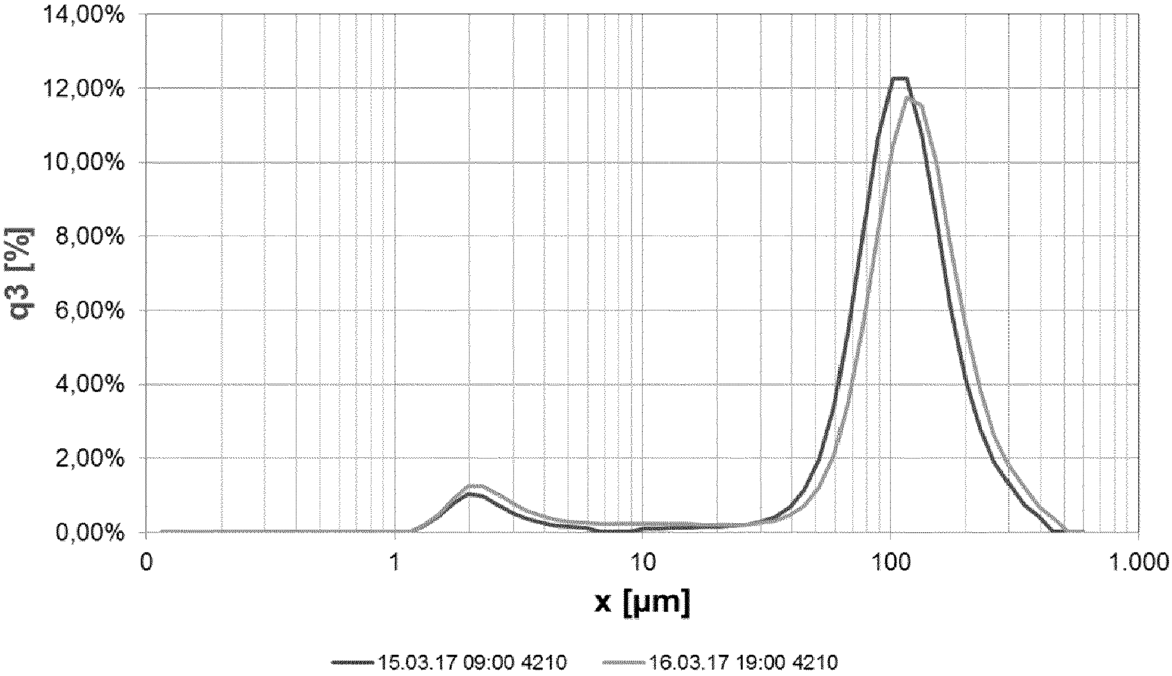


vor Rühren (dunkle Linie) nach 34 h Rühren (helle Linie)

Figur 5: Verfahrensschema zur Herstellung von DL,LD-Methionyl-methionin

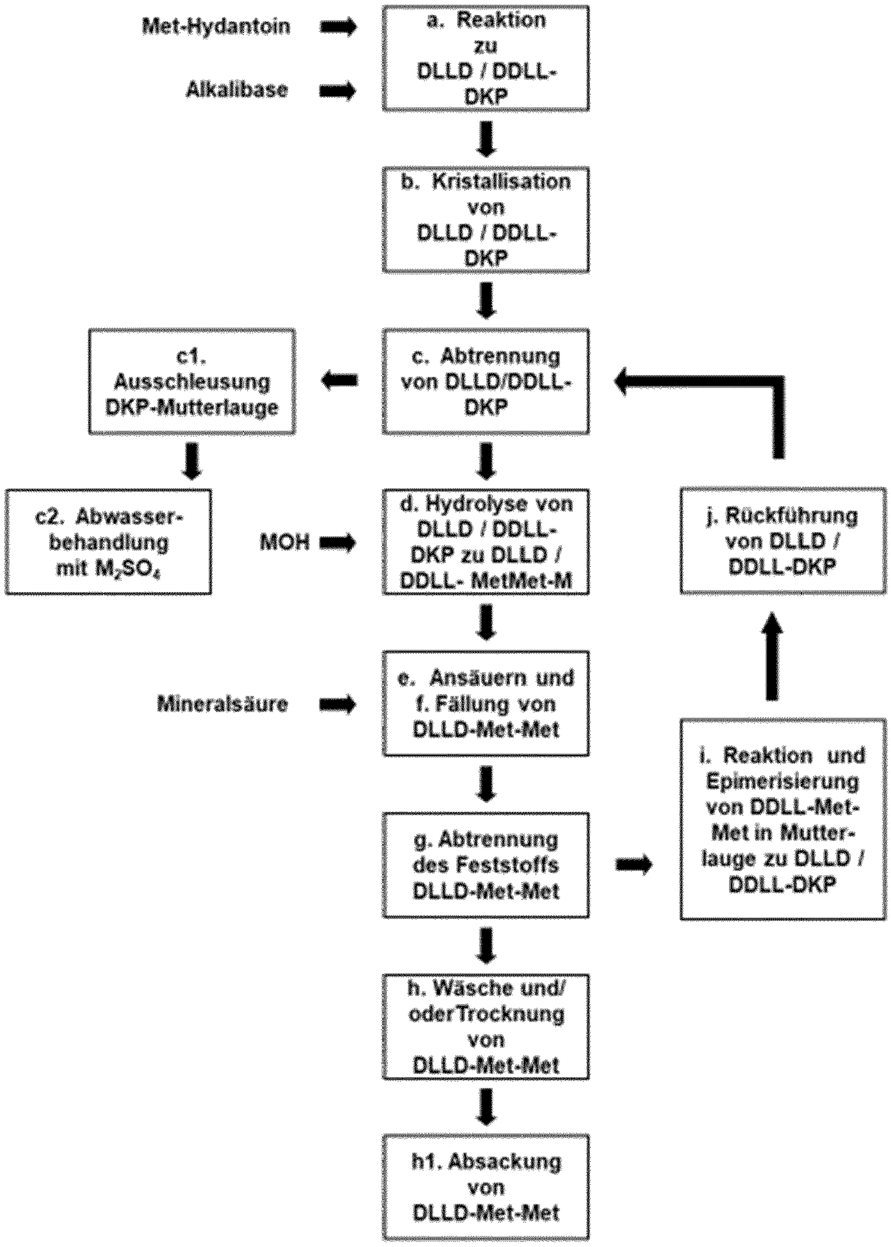

Europäisches Patentamt
European Patent Office
Office européen des brevets

# EUROPÄISCHER RECHERCHENBERICHT

Nummer der Anmeldung
EP 20 21 7861

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| A,D | WO 2010/043558 A1 (EVONIK DEGUSSA GMBH [DE]; KOBLER CHRISTOPH [DE] ET AL.) 22. April 2010 (2010-04-22) * Seite 21, Zeile 1 - Zeile 8; Beispiel 20 * ----- | 1-11 | INV. C07K5/062 A23K50/80 |
| | | | RECHERCHIERTE SACHGEBIETE (IPC) |
| | | | C07K C11C A23K |

Der vorliegende Recherchenbericht wurde für alle Patentansprüche erstellt

1

| Recherchenort | Abschlußdatum der Recherche | Prüfer |
|---|---|---|
| Den Haag | 11. Juni 2021 | Schleifenbaum, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 20 21 7861

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-06-2021

| Im Recherchenbericht angeführtes Patentdokument | Datum der Veröffentlichung | Mitglied(er) der Patentfamilie | Datum der Veröffentlichung |
|---|---|---|---|
| WO 2010043558 A1 | 22-04-2010 | BR PI0920882 A2 | 21-02-2017 |
| | | CA 2740663 A1 | 22-04-2010 |
| | | CL 2011000845 A1 | 26-08-2011 |
| | | CN 101720858 A | 09-06-2010 |
| | | CN 102186358 A | 14-09-2011 |
| | | CN 105053577 A | 18-11-2015 |
| | | CN 105061555 A | 18-11-2015 |
| | | DE 102008042932 A1 | 22-04-2010 |
| | | EP 2348881 A1 | 03-08-2011 |
| | | ES 2437602 T3 | 13-01-2014 |
| | | JP 5693457 B2 | 01-04-2015 |
| | | JP 6033254 B2 | 30-11-2016 |
| | | JP 2012505646 A | 08-03-2012 |
| | | JP 2014193176 A | 09-10-2014 |
| | | MY 155599 A | 13-11-2015 |
| | | PL 2348881 T3 | 31-03-2014 |
| | | RU 2011119472 A | 20-03-2013 |
| | | US 2010098801 A1 | 22-04-2010 |
| | | US 2013008384 A1 | 10-01-2013 |
| | | US 2013011514 A1 | 10-01-2013 |
| | | US 2015223495 A1 | 13-08-2015 |
| | | WO 2010043558 A1 | 22-04-2010 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010043558 A1
- EP 20217816


### In der Beschreibung aufgeführte Nicht-Patentliteratur


- **VON H. REHAGE et al.** *Chemical Engineering Science,* 2019, vol. 207, 258 ff